# EUROPÄISCHE PATENTSCHRIFT

(11) **EP 1 776 586 B1**
(45) Veröffentlichungstag und Bekanntmachung des Hinweises auf die Patenterteilung: **30.07.2008**
(21) Anmeldenummer: 05764146.6
(22) Anmeldetag: 07.07.2005
(51) Int. Cl.: G01N 33/552, G01N 33/548

(54) **TRÄGERPLATTE ZUR DURCHFÜHRUNG FUNKTIONELLER TESTS AN BIOLOGISCHEN ZELLEN SOWIE VERFAHREN ZUR BESCHICHTUNG DER TRÄGERPLATTE**
CARRIER FOR CARRYING OUT FUNCTIONAL TESTS ON BIOLOGICAL CELLS AND METHOD FOR COATING SAID CARRIER
PLAQUE SUPPORT POUR REALISER DES TESTS FONCTIONNELS SUR DES CELLULES BIOLOGIQUES ET PROCEDE POUR ENDUIRE CETTE PLAQUE-SUPPORT

(30) Priorität: 10.08.2004 DE 102004039628
(43) Veröffentlichungstag der Anmeldung: 25.04.2007
(73) Patentinhaber: NMI NATURWISSENSCHAFTLICHES UND MEDIZINISCHES INSTITUT AN DER UNIVERSITÄT TÜBINGEN, 72770 Reutlingen (DE)
(72) Erfinder: STEUER, Heiko, 72793 Pfullingen (DE); TEMPLIN, Markus, 72074 Tübingen (DE); KANZOK, Britta, 73728 Esslingen (DE); KUSCHEL, Cornelia, 72116 Mössingen (DE); ANGRES, Brigitte, 72793 Pfullingen (DE)
(74) Vertreter: Laufer, Gabriele
(86) Internationale Anmeldenummer: PCT/EP2005/007334
(87) Internationale Veröffentlichungsnummer: WO 2006/018072

(56) Entgegenhaltungen:
- EP-A- 0 366 241
- WO-A-95/25116
- US-A1- 2003 129 296
- US-A1- 2004 081 979
- TONKINSON JOHN L ET AL: "Nitrocellulose: a tried and true polymer finds utility as a post-genomic substrate." FRONTIERS IN BIOSCIENCE : A JOURNAL AND VIRTUAL LIBRARY. 1 JAN 2002, Bd. 7, 1. Januar 2002 (2002-01-01), Seiten c1-12, XP008054957 ISSN: 1093-4715
- MANDENIUS C F ET AL: "OPTICAL SURFACE METHODS FOR DETECTION OF NUCLEIC ACID BINDING" ANALYTICAL LETTERS, Bd. 22, Nr. 15, 1989, Seiten 2961-2974, XP008054940 ISSN: 0003-2719

## Beschreibung

Die vorliegende Erfindung betrifft ein Verfahren zum Beschichten einer Trägerplatte zur Durchführung funktioneller Tests an biologischen Zellen, eine Trägerplatte zur Durchführung funktioneller Tests an biologischen Zellen sowie die Verwendung entsprechender Trägerplatten zur Durchführung funktioneller Tests an biologischen Zellen.

Im Bereich der biologischen Grundlagenforschung sowie in der angewandten Biotechnologie, wie bspw. dem Wirkstoff-Screening oder der Diagnostik, werden Trägerplatten, z.B. aus Glas oder Kunststoff verwendet, mittels derer eine Vielzahl von Untersuchungen wie Interaktionen zwischen biologischem Material und chemischen/biochemischen Substanzen untersucht werden können.

Dazu werden die Trägerplatten in der Regel beschichtet, bspw. mit Poly-L-Lysin, über das biologisches Material, wie biologische Zellen, an die Oberfläche der Trägerplatte fixiert wird. An den fixierten Zellen können dann funktionelle Tests durchgeführt werden. Derart ausgestaltete Trägerplatten werden auch als Biochips bezeichnet. Der Beschichtung der Trägerplatte mit Materialien verschiedenster Art kommt dabei eine entscheidende Rolle zu, da von dieser sämtliche darauf folgenden Schritte, wie das Aufbringen von Biomolekülen, die Besiedelung mit biologischen Zellen sowie die Reproduzierbarkeit des funktionellen Tests mittels des fertigen Biochips, abhängen.

Aus der WO 02/02226 A2 ist ein Verfahren zum Beschichten einer Trägerplatte bekannt, bei dem Polylysin auf eine aldehydaktivierte Oberfläche der Trägerplatte mittels konventioneller Methoden der Inkjet-Technologie oder des Kontaktprintings gespottet wird. In einem daran anschließenden Schritt werden extrazelluläre Matrix(ECM-)proteine kovalent an das zuvor aktivierte Polylysin gebunden. Um eine unspezifische Bindung von Zellen an die aldehyd-aktivierte Oberfläche zu vermeiden, werden noch freie Aldehydgruppen innerhalb und vor allem außerhalb der Mikrospots abgesättigt. Dieselbe Behandlung erfolgt auch dann, wenn Proteine direkt an aldehydaktivierte Oberflächen gebunden werden. Der Nachteil des Verfahrens besteht darin, dass die Absättigung der Aldehydgruppen nicht ausreicht, um eine unspezifische Bindung von Zellen völlig zu verhindern.

Aus US 6,548,263 B1 ist ein Verfahren bekannt, das solch eine unspezifische Bindung von Zellen an Bereiche außerhalb der mit Biomolekülen beladenen Mikrospots zu verhindern versucht. Dabei wird eine Trägerplatte aus Glas, Kunststoff oder Silikon chemisch modifiziert, z.B. mit Aminosilan (3-aminopropyltrimethoxysilan). Proteine, die auf diese Oberfläche gespottet werden, können entweder direkt oder über hetero-bi-funktionelle Gruppen an die reaktiven Gruppen des Aminosilans gebunden werden. Bevor die Bindung der Biomoleküle erfolgt, wird eine zellabweisende, hydrophobe Beschichtung in Bereichen außerhalb der Mikrospots vorgenommen, um eine unspezifische Bindung von Zellen an diese reaktiven Gruppen zu verhindern. Bei dem hier offenbarten Verfahren handelt es ich um ein aufwändiges, mehrstufiges Verfahren. Ein weiterer Nachteil der in der US 6,548,263 beschriebenen Methode ist, dass dieselbe Stelle doppelt kongruent bespottet wird, was in der Praxis sehr schwierig ist, da ein Anfahren desselben Mikrospots äußerste Präzision des Gerätes erfordert, die mit den meisten Arrayern nicht gegeben ist.

Aus der US 2002/0019018 A1 ist eine mit Nitrocellulose beschichtete Trägerplatte in Form eines Glasobjektträgers bekannt, wobei über die Nitrocellulose monoklonale Antikörper an die Trägerplatte fixiert sind. Einzelheiten über das Beschichtungsverfahren werden nicht offenbart.

Im Handel sind ferner Glasträger erhältlich, die eine Nitrocellulose-Oberfläche aufweisen. Diese Oberfläche kann durch entsprechende Reagenzien abgesättigt werden, um die unspezifische Adhäsion an Zellen zu vermeiden. Die Nitrocellulose-Schicht hat jedoch den Nachteil, dass daran zu fixierendes biologisches Material, wie bspw. ECM (Extrazelluläre Matrix)-Proteine, wegen der hohen Saugfähigkeit der kompakten Nitrocellulose-Schicht schwammartig aufgesaugt werden, damit in den Tiefen der Schicht verloren gehen und einer anschließenden Besiedelung mit biologischen Zellen nicht mehr zur Verfügung stehen. Die Adhäsion der Zellen ist relativ gering, obgleich kostenaufwändig große Mengen von ECM-Proteinen eingesetzt werden. Um eine für die Zelladhäsion effektive, den Zellen zugängliche Proteinschicht zu generieren, müssen unnötig hohe Konzentrationen von Proteinen aufgebracht werden, was technisch mit Mikroarrayern, v.a. Inkjet Arrayern, schwierig ist.

Aufgabe der vorliegenden Erfindung ist es deshalb, ein Verfahren zur Beschichtung einer Trägerplatte bereitzustellen, mit dem die Nachteile aus dem Stand der Technik vermieden werden. Insbesondere soll eine Beschichtung ermöglicht werden, die eine sichere und gute Fixierung von Biomaterial ermöglicht, die ferner eine einfache Absättigung unspezifischer Adhäsionsstellen für Zellen außerhalb der Biomolekülspots zulässt und die eine gute Adhäsion mit bzw. von biologischen Zellen über gebundene Biomoleküle gewährleistet.

Diese Aufgabe wird durch ein eingangs genanntes Verfahren gelöst, das folgende Schritte aufweist:
(a) Bereitstellen einer Trägerplatte mit einer ersten Schicht, die Poly-L-Lysin oder Derivate davon aufweist, und
(b) Beschichten der mit der ersten Schicht beschichteten Trägerplatte mit Nitrocellulose oder Derivaten davon zur Bildung einer rauen Nitrocellulose-Schicht mit eines Schichtdicke von ca. 100 bis ca. 1200 nm.

Die der Erfindung zu Grunde liegende Aufgabe wird hiermit vollkommen gelöst. Die Erfinder haben nämlich erkannt, dass insbesondere Nitrocellulose nach einer vorhergehenden Beschichtung der Trägerplatte mit einer ein Polykation aufweisenden Schicht aufgebracht werden kann. Dadurch wird eine gute Haftung der Nitrocellulose gewährleistet und ein Substrat für das Aufbringen von Biomolekülen, wie bspw. Komponenten der extrazellulären Matrix, bereitgestellt, das in einem weiteren Schritt mit den zu untersuchenden biologischen Zellen besiedelt werden kann. Die so hergestellte Trägerplatte vereinigt die guten Eigenschaften einer mit Nitrozellulose beschichteten Trägerplatte, nämlich die einfache Absättigung unspezifischer Bindestellen sowie die einfache Herstellung der Trägerplatte.

Unter "Trägerplatte" wird hierbei jede platten- oder plättchenähnliche Vorrichtung verstanden, die für einen Einsatz zur Durchführung funktioneller Tests an biologischen Zellen geeignet ist, wie bspw. Glasplatten, Kunststoffplatten, insbesondere Kunststoffplatten aus Polystyrol und/oder Silikon, bspw. in Gestalt von Multiwellplatten.

Bevorzugt ist dabei, wenn in Schritt (a) eine Trägerplatte mit einer Schicht bereitgestellt wird, die einen Stoff aufweist, der ausgewählt ist aus der Gruppe umfassend Poly-L-Lysin, Poly-D-Lysin, Polyimid, Aminosilan, oder Derivate davon.

In dem erfindungsgemäßen Verfahren hat sich eine Beschichtung mit Poly-L-Lysin als besonders geeignet herausgestellt.

Bevorzugt ist ferner, wenn die Beschichtung in Schritt (b) derart erfolgt, dass eine dünne und raue Schicht aus Nitrocellulose ausgebildet wird.

Durch die dünne Schicht der Nitrocellulose wird gewährleistet, dass der Diffusionsraum, der von der Lösung zurückgelegt wird, klein gehalten ist. Im Gegensatz zu dicken oder kompakten Nitrocellulose-Schichten, bei welchen die Ausbreitung des Biomoleküls - und damit auch der Durchmesser des Biomolekülspots auf der Trägeroberfläche - von der Reagenzienlösung abhängt, kann daher der erfindungsgemäße Träger auch für quantitative Zellzahlbestimmungen eingesetzt werden. Ferner können die erfindungsgemäßen Träger bei der Durchlichtmikroskopie eingesetzt werden, da bei ihnen - im Gegensatz zu Trägern mit dickeren Nitrocellulose-Schichten - das Licht in dieser Schicht nicht gestreut wird und dadurch kein diffuses Bild entsteht. Dadurch können bei dieser Mikroskopieart Zellen auf dem erfindungsgemäßen Träger einfach und zuverlässig beobachtet werden.

Unter "dünn" wird hierbei jede Schichtdicke verstanden, die bei den so gebildeten Trägerplatten noch eine Mikroskopie mit Durchlicht zulässt, die zumindest aber kleiner als ca. 15.000 nm ist.

Unter "rau" wird dabei jede Schicht verstanden, deren Oberfläche - im Vergleich zu der Oberfläche einer "glatten Schicht" - kein ebenmäßiges Erscheinungsbild aufweist. Dadurch umfasst die raue Schicht insgesamt eine größere Oberfläche als eine vergleichbare glatte Schicht.

Dabei ist es bevorzugt, wenn die Beschichtung in Schritt (b) durch Aufbringen einer Methanol-Nitrocellulose-Lösung und anschließendem Verdunstenlassen des Methanols erfolgt.

Nach einer bevorzugten Weiterbildung des erfindungsgemäßen Verfahrens erfolgt das Aufbringen der Methanol-Nitrocellulose-Lösung durch Besprühen der Trägerplatte.

Beide Verfahren haben den Vorteil, dass auf einfache Art und Weise eine raue Nitrocelluloseschicht ausgebildet wird, nachdem das Methanol verdunstet ist. Die dadurch gebildete Nitrocellulose-Schicht weist eine weitgehend raue Oberfläche auf.

Dabei wird eine größere Oberfläche als bei glatten Nitrocelluloseschichten oder nur chemisch aktivierten Oberflächen bereitgestellt. Somit kann in einem weiteren Schritt mehr Biomaterial, wie bspw. extrazelluläre Matrixproteine, aufgebracht bzw. immobilisiert werden und damit ein besseres Substrat für die Besiedelung mit biologischen Zellen bereitgestellt werden, da dadurch vergleichsweise mehr Biomoleküle pro Fläche für die Bindung von biologischen Zellen zur Verfügung stehen.

Alternativ erfolgt das Aufbringen der Methanol-Nitrocellulose-Lösung durch Eintauchen der Trägerplatte in die Lösung.

Nach dem Eintauchen wird die Trägerplatte herausgezogen und an der Luft getrocknet. Dieses Verfahren hat den Vorteil, dass hierbei eine relativ glatte Nitrocelluloseschicht gebildet werden kann.

Die genannten Beschichtungsmaßnahmen haben den Vorteil, dass äußerst dünne Schichten von Nitrocellulose ausgebildet werden können, mit einer Schichtdicke von ca. 100 bis ca. 1200 nm. Damit wird ein Schwammeffekt, bei dem Biomaterial in die tieferen Schichten der Nitrocelluloseschicht hineingesaugt wird und damit verloren geht, vermieden, wodurch der Großteil des Biomaterials für die Bindung an Zellen zur Verfügung steht.

Für eine Vielzahl von funktionellen Tests wird das Biomaterial in Form von Tropfen auf die Trägerplatte aufgebracht. In diesem Zusammenhang ist die Ausbildung von dünnen Nitrocellulose-schichten besonders vorteilhaft, da wegen der geringen Diffusionsstrecken des in Lösung befindlichen Biomaterials der Durchmesser der resultierenden sog. Mikrospots gut kontrollierbar ist. Unter "Mikrospots" werden demnach punktförmige Ausbreitungen des Biomaterials auf der Trägerplattenoberfläche verstanden. In funktionellen Tests, in denen Zellzahlen pro Mikrospot ermittelt werden, werden damit zuverlässigere und besser reproduzierbare Ergebnisse erzielt.

Das Besprühen der Trägerplatte erfolgt dabei mittels konventioneller Techniken, bspw. unter Verwendung einer Druckluftpistole (Airbrush-Pistole) oder dem Vernebeln der Methanol-Nitrocellulose-Lösung über der mit einer Polykation-Schicht beschichteten Trägerplatte mit einem entsprechenden Gerät. Gemäß der alternativen Vorgehensweise wird der mit einer Polykation-Schicht beschichtete Träger in ein Gefäß eingetaucht, das Methanol-Nitrocellulose-Lösung enthält.

Nach einer erfindungsgemäßen Variante wird in Schritt (a) eine unbeschichtete Trägerplatte mit einer Schicht beschichtet, die Poly-L-Lysin, oder Derivate davon aufweist.

Diese Maßnahme hat den Vorteil, dass eine Vielzahl von kommerziell erhältlichen Trägerplatten verwendet werden kann, die noch nicht mit einer Schicht vorbeschichtet sind, die zumindest einen Wasserstoffbrücken-Donor und/oder zumindest ein Polykation aufweist. Der Anwender des erfindungsgemäßen Verfahrens hat damit bei der Wahl der passenden Trägerplatte noch größere Freiheiten, wonach bspw. auch Mikrotiterplatten verwendet werden können.

Erfindungsgemäß bevorzugt ist es ferner, wenn ein weiterer Schritt (c) erfolgt, in dem Biomoleküle auf die beschichtete Trägerplatte aufgebracht bzw. immobilisiert werden.

Unter Biomolekülen werden jedwede biologischen Substanzen verstanden, wie Proteine, Kohlenhydrate, Lipide etc. Insbesondere kommen sog. Fängermoleküle, wie Antikörper, Zelloberflächenproteine, Rezeptoren, Liganden, Lektine, Antigene und Allergene etc. in Frage.

Dabei ist es erfindungsgemäß bevorzugt, wenn die Biomoleküle ausgewählt sind aus der Gruppe bestehend aus: Protein, insbesondere Protein der extrazellulären Matrix, wie Fibronektin, Kollagen, Laminin, Thrombospondin, Elastin, Tenascin, Vitronektin; Kohlenhydrate, insbesondere Kohlenhydrate der extrazellulären Matrix, wie Glykosaminoglykane; Proteoglykane; Lipide.

Mit den auf diese Weise gefertigten Trägerplatten, können bspw. Bindungstests durchgeführt werden, also Tests, in welchen bspw. untersucht wird, welche Zellarten oder wie viele Zellen an die jeweiligen Biomoleküle gebunden haben.

Mit dem Aufbringen von Komponenten der extrazellulären Matrix auf die mit bspw. einer ersten Polykation-Schicht und mit Nitrocellulose beschichtete Trägerplatte wird eine weitgehend natürliche Umgebung für die Zellen bereitgestellt, so dass der ursprüngliche biologische funktionelle Status dieser Zellen aufrechterhalten wird. So ist nämlich bekannt, dass die extrazelluläre Matrix Zellfunktionen und -differenzierungen mit kontrolliert. Durch diese Maßnahme wird also *in vitro* eine *in vivo*-ähnliche Situation geschaffen und damit bspw. bei einem Wirkstoff-Screening eine höhere Wahrscheinlichkeit für das schnelle Auffinden von *in vivo* wirksamen Substanzen erzielt. Dem entgegen werden in den meisten zellbasierenden Screening-Assays lediglich Kunststoffoberflächen zur Zellbesiedelung verwendet, was häufig zur Dedifferenzierung und dem Verlust von Zellfunktionen führt.

Es ist ferner bevorzugt, wenn bei dem erfindungsgemäßen Verfahren ein weiterer Schritt (c') erfolgt, in dem Testsubstanzen auf die beschichtete Trägerplatte aufgebracht werden. Die Testsubstanzen sind dabei vorzugsweise ausgewählt aus der Gruppe bestehend aus: pharmazeutische Präparate; Antikörper; Substanzen, die die Eigenschaften der biologischen Zellen beeinflussen; Botenstoffe; Wachstumsfaktoren; Antigene; Allergene.

Durch das In-Kontakt-Bringen von zu immobilisierenden Zellen mit den genannten Testsubstanzen können die Zellen gezielt stimuliert oder allgemein in ihren Eigenschaften beeinflusst, bzw. das Potenzial einer solchen Testsubstanz zur Beeinflussung der biologischen Zellen untersucht werden. Gleichermaßen kann bspw. bei der Verwendung von gegen Tumormarker gerichteten Antikörpern als Testsubstanz eine Trägerplatte geschaffen werden, die zum Krebszell-Screening eingesetzt werden kann.

Weiter ist es bevorzugt, wenn bei dem erfindungsgemäßen Verfahren die Biomoleküle und/oder Testsubstanzen mittels kontaktlosem oder Kontakt-vermitteltem Drucken erfolgt.

Dabei ist insbesondere bevorzugt, wenn das kontaktlose Drucken mittels der ink-jet-Technologie, bzw. über piezo-elektrisches Drucken, und das Kontakt-vermittelte Drucken durch das Pin und Ring Verfahren oder das Split-Pin Verfahren erfolgt.

Verfahren zur Durchführung der ink-jet Technologie sind bspw. in den US-Patentschriften mit den Nummern 5,233,369 und 5,486,855 offenbart. Eine allgemeine Übersicht über Verfahren zum Kontakt-vermittelten Drucken wie auch zum kontaktlosem Drucken findet sich bspw. in "Microarray Biochip Technology", Schena, M, Ed., 2000.

Durch das kontaktlose Drucken wird bspw. sichergestellt, dass stets gleichmäßige Mengen von Biomolekülen oder Testsubstanzen aufgebracht werden und keine Verschleppung dieses Materials erfolgt. Dem gegenüber kann es beim herkömmlichen sog. Kontakt-Printing mit einem Nadeldrucker zu einer großen Variation der Mengen von aufgebrachtem Material von Auftragung zu Auftragung kommen. Auch das Problem der Verschleppung von Protein beim Erstellen von Mikrospots wird dabei häufig beobachtet. Diesen Nachteilen wird jedoch durch die bevorzugten Maßnahmen vorgebeugt.

Ferner ist es bevorzugt, wenn bei dem erfindungsgemäßen Verfahren die Biomoleküle und/oder Testsubstanzen in einem Druckpuffer bereitgestellt werden, der Trehalose in einer Endkonzentration von 0,1 bis 5 % (w/v), vorzugsweise 0, 5 % (w/v), und NP40 in einer Endkonzentration von 0,00001 bis 0,1 % (v/v), vorzugsweise 0,0003 bis 0,005 % (v/v) enthält.

Hierbei ist jedoch nicht ausgeschlossen, dass auch andere, kommerziell erhältliche Druckpuffer eingesetzt werden können, wie bspw. der Druckpuffer "Protein Printing Buffer Arraylt"^{™} der Firma Telechem, USA.

Wie die Erfinder herausgefunden haben, wird durch die Verwendung eines solchen Druckpuffers im Rahmen des piezo-elektrischen Druckens die Ausbildung von regelmäßigen Mustern bzw. regelmäßigen Mikrospots sichergestellt und das Entstehen von sogenannten unerwünschten Satellitenspots oder unregelmäßigen Mustern weitgehend vermieden. Die durchzuführenden funktionellen Tests liefern damit zuverlässigere und reproduzierbare Ergebnisse.

Nach einer bevorzugten Weiterbildung des erfindungsgemäßen Verfahrens erfolgt ein weiterer Schritt (d), in dem die vorbehandelte Trägerplatte mit einer Proteinlösung, vorzugsweise einer 5 %igen (w/v) BSA-PBS-Lösung, inkubiert wird. Hierbei können aber auch andere sog. Blockpuffer eingesetzt werden, wie bspw. der kommerziell erhältliche Blockpuffer "StabilGuard" der Firma SurModics.

Diese Maßnahme hat den Vorteil, dass unspezifische Bindungsstellen im mit Biomaterial beschichteten Mikrospot, bspw. den extrazellulären Matrixproteinen, und unspezifische Zell-Bindungsstellen der Nitrocellulose außerhalb der Mikrospots auf dem Träger abgesättigt werden. Dabei hat sich die bevorzugte Konzentration von BSA (Rinderserumalbumin) in PBS (Phosphat gepufferte Saline) als eine solche herausgestellt, die eine optimale Absättigung derartiger Bindestellen gewährleistet.

Ferner ist es bevorzugt, wenn bei dem erfindungsgemäßen Verfahren ein weiterer Schritt (e) erfolgt, in dem biologische Zellen auf die vorbehandelte Trägerplatte aufgebracht werden.

Mit derart vorgefertigten Trägerplatten können weitere Tests durchgeführt werden. So kann bspw. nach Zugabe von löslichen Substanzen untersucht werden, wie und auf welche Substanzen die auf der Trägerplatte fixierten Zellen reagieren.

Weiter ist es bevorzugt, wenn die in Schritt (a) bereitgestellte Trägerplatte aus einem Material gefertigt ist, das ausgewählt ist aus der Gruppe bestehend aus: Glas; Kunststoff, insbesondere Polystyrol und/oder Silikon.

Bei dieser Maßnahme ist von Vorteil, dass die durchzuführenden funktionellen Tests mikroskopisch, insbesondere im Rahmen von Durchlichtmessungen ausgewertet werden können, wobei sich auf Glas und Kunststoff in bevorzugter Weise Poly-L-Lysin und Nitrocellulose beschichtete Oberflächen vorsehen lassen, auf denen das Biomaterial und/oder Testsubstanzen leicht aufgebracht bzw. immobilisiert werden können.

Gegenstand der vorliegenden Erfindung ist ferner eine Trägerplatte zur Durchführung funktioneller Tests an biologischen Zellen, bestehend aus einer Basisplatte, die mit einer ersten Schicht beschichtet ist, die Poly-L-Lysin, oder Derivate davon aufweist.

Bei diesen Platten ist von Vorteil, dass durch die erste Polykation-Schicht eine gute Haftung der Nitrocellulose sichergestellt wird.

Bei der erfindungsgemäßen Trägerplatte ist vorzugsweise eine dünne Schicht aus Nitrocellulose vorgesehen und insbesondere eine Nitrocellulose-Schicht mit einer Schichtdicke von ca. 100 bis ca. 1200 nm.

Hiermit wird sichergestellt, dass der oben erwähnte Schwammeffekt weitgehend vermieden wird.

Je nach Aufbringungs-Verfahren der Nitrocellulose-Schicht ist dabei die Oberfläche glatter oder rauer. Insbesondere in letzterem Fall wird auf Grund der Rauhigkeit eine große Oberfläche für das anzubringende Biomaterial, wie bspw. extrazelluläre Matrixproteine, bereitstellt.

Weiter ist es bevorzugt, wenn bei der Trägerplatte auf die Schicht aus Nitrocellulose eine Schicht aus Biomolekülen und/oder Testsubstanzen aufgebracht ist, wobei die Biomoleküle vorzugsweise aus der Gruppe ausgewählt sind, bestehend aus: Proteine, insbesondere Proteine der extrazellulären Matrix, wie Fibronektin, Laminin, Thrombospondin, Kollagen, Elastin, Tenascin, Vitronektin; Kohlenhydrate, insbesondere Kohlenhydrate der extrazellulären Matrix, wie Glukosaminoglykane; Proteoglykane; Lipide. Die Testsubstanzen sind vorzugsweise ausgewählt aus der Gruppe bestehend aus: pharmazeutische Präparate; Antikörper; Substanzen, die die Eigenschaften der biologischen Zellen beeinflussen; Botenstoffe; Wachstumsfaktoren; Antigene; Allergene.

Durch diese Maßnahmen wird bei der Auswahl von ECM-Komponenten eine Trägerplatte bereitgestellt, die für die zu immobilisierenden Zellen eine weitgehend natürliche, in vivo-ähnliche Umgebung schaffen, so dass Dedifferenzierungen der Zellen und Artefaktmessungen im Rahmen der funktionellen Tests vermieden werden können. Ferner wird bei der Auswahl von Fängermolekülen oder pharmazeutischen Präparaten eine Trägerplatte bereitgestellt, die zum Krebszell-Screening oder auch zur Stimulierung von Zellen verwendet werden kann und damit ein wichtiges Instrument für die Diagnostik und das Wirkstoff-Screening darstellt.

Nach einer bevorzugten Weiterbildung sind auf die Biomoleküle und/oder Testsubstanzen biologische Zellen aufgebracht.

Dadurch wird eine bereits testfertige Trägerplatte in Form eines Biochips zur direkten Verwendung bereitgestellt.

Weiter ist bevorzugt, wenn die Basisplatte der erfindungsgemäßen Trägerplatte aus einem Material gefertigt ist, das ausgewählt ist aus der Gruppe bestehend aus: Glas; Kunststoff, insbesondere Polystyrol und/oder Silikon.

Diese Maßnahme hat den Vorteil, dass einerseits auf vorteilhafte Weise mittels Standardverfahren eine beschichtete Oberfläche vorgesehen werden kann und andererseits die Auswertung der funktionellen Tests auch mittels mikroskopischer Durchlichtmessungen ermöglicht wird.

Ein weiterer Gegenstand der vorliegenden Erfindung ist die Verwendung der vorstehend beschriebenen Trägerplatte bzw. der Trägerplatte, hergestellt nach dem Verfahren gemäß einem der Ansprüche 1 bis 14, oder einer Trägerplatte gemäß einem der Ansprüche 15 bis 20 zur Durchführung funktioneller Tests an biologischen Zellen.

Die vorliegende Erfindung wird nun anhand von Ausführungsbeispielen und Abbildungen näher erläutert, aus denen sich weitere Vorteile und Eigenschaften ergeben. Es zeigen:
- Fig. 1: eine Ausführungsform der erfindungsgemäßen Trägerplatte;
- Fig. 2: die differenzielle Adhäsion von drei Zelllinien auf mit verschiedenen ECM-Proteinen beschichteten Trägerplatten [(A) absolute Zellzahlen; (B) relative Zellzahlen].

### Beispiel 1: Beschichtung der Trägerplatte mit Nitrocellulose

Als Trägerplatten wurden bereits mit Poly-L-Lysin beschichtete Objektträger (Poly-Prep-Slides, Sigma P0425) verwendet. Die Beschichtung mit Nitrocellulose-Lösung kann auf zweierlei Arten erfolgen:
(A) Beschichten im Sprühverfahren
   Die Objektträger wurden der Länge nach nebeneinander senkrecht an eine Schiene gestellt. Die Nitrocellulose-Lösung (2,5 mg/ml Methanol) wurde mit einer Airbrush-Pistole, die im Abstand von 18 cm langsam parallel zu den Objektträgern entlanggeführt wird, auf diese aufgebracht. Das Besprühen wurde vierzehn Mal wiederholt, wobei das Methanol nach jedem Sprühvorgang innerhalb kurzer Zeit verdunstet.
(B) Beschichten im Tauchverfahren
   Die Objektträger wurden senkrecht hängend mit der Beschriftungsfläche an eine Schiene geklemmt. Mithilfe einer entsprechenden motorgetriebenen Vorrichtung wurden die Objektträger in ein Gefäß mit Nitrocellulose-Lösung (20 mg/ml Methanol) abgesenkt. Nach 10 min wurden die Objektträger langsam (10 cm/min aus der Lösung gezogen und hängend 10 min getrocknet. Zur Kontrolle der Gleichmäßigkeit der Nitrocelluloseschicht wurden die wie beschrieben beschichteten Objektträger mit hoher Verstärkung (z.B. Gain 90) bspw. mittels eines GMS 418 Arrayer Scanners gescannt. Ungleichmäßig beschichtete Objektträger wurden aussortiert und nicht verwendet.

### Beispiel 2: Beschichtung der Trägerplatte mit Biomolekülen.

Nachfolgende Schritte wurden steril bzw. im Reinraum durchgeführt.

Zur Beschichtung einer wie nach Beispiel 1 vorbehandelten Trägerplatte mit Biomolekülen (hier als Beispiel: ECM-Moleküle) wurden zwei verschiedene Druckpuffer benötigt: Puffer I (0,5 % (w/v) Trehalose mit 0,05 % (v/v) NP40 in PBS: für alle in Beispiel 5 beschriebenen ECM Proteine außer für Kollagene) und Puffer II (0,5 % (w/v) Trehalose mit 0,0003 % (v/v) NP40 in PBS: für Kollagene). Den Druckpuffern wurde BSA-Tamra (Rinderserum Albumin, das kovalent an den Fluoreszenzfarbsoff Carboxytetramethylrhodamin gekoppelt wurde) in einer Endkonzentration von 1µg/ml beigemischt. Die Zugabe des BSA-Tamra zur Biomoleküllösung ermöglichte es, die Proteinlösung nach Auftrag auf die Trägerplatte über einen Laserscanner sichtbar zu machen und damit die Korrektheit des Mikrospotmusters direkt nach dem Druck zu kontrollieren. Nach dem Waschen der Objektträger mit Sättigungslösung (siehe Beispiel 3) diente BSA-Tamra, das sich ebenso wie die anderen Biomoleküle im Druckpuffer an die Trägeroberfläche adsorbierte, zur relativen Quantifizierung des adsorbierten Proteins an die Trägerplatte. Die Druckpuffer wurden doppelt konzentriert angesetzt und mit den Biomolekülen, die in doppelter Konzentration der gewünschten Endkonzentration in PBS vorliegen, 1:2 gemischt. Die mit Druckpuffer versetzten ECM-Moleküle wurden 15 min bei 4°C bei 15000 g (z.B. Eppendorf-Zentrifuge 5810R mit Rotor für Eppendorf-Röhrchen: 12000 rpm) zentrifugiert und in eine Platte mit 384 Vertiefungen (Genetix X6003; 20 bis 40 µl pro Vertiefung) pipettiert. Die Platte wurde nochmals für 10 min bei 4°C bei 1500 g (z.H. Hettich-Zentrifuge mit Rotor für Multi-Well-Platten, 4000 rpm) zentrifugiert.

Die Objektträger wurden mit dem BioChipArrayer (Packard) gemäß den Anweisungen des Herstellers mit den Biomolekülen beschichtet bzw. bedruckt (z.B. ein Array mit 8x8 Mikrospots, 12 Arrays pro Objektträger). Wichtig war hierbei das Einhalten der Waschprozeduren des BioChip Arrayers wie vom Hersteller angegeben.

Zur Überprüfung der korrekten Beschichtung der Trägerplatte mit den Biomolekülen wurden die Objektträger mit niedriger Verstärkung (z.B. Gain 30) gescannt. Die Mikrospots erschienen im linearen Bereich in den Falschfarben blau bzw. grün. Sowohl das Druckmuster als auch die relativen Mengen des gespotteten Proteins pro Mikrospot konnten so indirekt über die Quantifizierung der Fluoreszenz des beigemischten BSA-Tamra überprüft werden. Nach dem Scannen wurden die Objektträger bei 4°C in geschlossenen Behältern gelagert.

### Beispiel 3: Absättigen unspezifischer Bindungsstellen der Trägerplatte

Nachfolgende Schritte wurden steril bzw. im Reinraum durchgeführt.

Die wie nach den Beispielen 1 und 2 vorbereiteten Objektträger wurden nach einer Lagerung von ca. zwei Tagen (oder länger s.u.) eine Stunde bei Raumtemperatur akklimatisiert mit StabilGuard (SurModics SG01B04) oder BSA-Lösung (BSA von Roth T844; 5 % (w/v) in PBS; 1 h bei 50°C hitzeinaktiviert) "geblockt" und bei Raumtemperatur getrocknet. Das "Blocken", also das Absättigen der unspezifischen Bindungsstellen, kann auf verschiedene Arten erfolgen:
(A) Absättigen unspezifischer Bindungsstellen mithilfe eines Sprühverfahrens
   Hier wurden die Objektträger waagrecht liegend mit einem Sprühnebel benetzt, bis sie gleichmäßig von einem Feuchtigkeitsfilm bedeckt waren. Die Sprühflasche wurde ca. 30 cm vor den Objektträgern senkrecht gehalten. Nach einer Stunde bei Raumtemperatur wurden die Objektträger mit der bedruckten Seite nach unten schräg stehend 30 min getrocknet.
(B) Absättigen unspezifischer Bindungsstellen im Tauchverfahren
   Hierbei wurden die Objektträger mit dem Beschriftungsfeld nach oben in eine Küvette mit Blocklösung getaucht, nach einer Stunde herausgezogen und mit der bedruckten Seite nach unten schräg stehend 30 min getrocknet. Anschließend wurden sie bei 4°C in geschlossenen Behältern gelagert.
   Zur Kontrolle der relativen Mengen der adsorbierten ECM-Proteine in den Mikrospots, wurden die geblockten Objektträger gescannt und die Intensitäten der die Fluoreszenz der Mikrospots, herrührend von beigemischtem BSA-Tamra zur gespotteten ECM-Proteinlösung, mit einem Quantifizierungsprogramm (z.B. Imagene, BioDiscovery) bestimmt.
   Alternativ hierzu können die Objektträger nach längerer Lagerungszeit als nur 2 Tage erst unmittelbar vor der Besiedelung mit biologischen Zellen geblockt werden. Hierbei wurden die ungeblockt gelagerten Objektträger 1 h bei Raumtemperatur akklimatisiert und direkt vor der Besiedelung wahlweise auf zwei verschiedene Arten geblockt:
(C) Blocken im Tauchverfahren
   Die Objektträger wurden mit dem Beschriftungsfeld nach oben in eine Küvette mit Blocklösung getaucht, nach einer Stunde herausgezogen, einmal in PBS-Lösung getaucht und feucht mit einem Pro-Plate-Kultivierungsaufsatz (Grace BioLabs, USA) versehen. Die Kavitäten wurden sofort mit je 200 µl PBS gefüllt.
(D) Blocken im Kultivierungsaufsatz
   Die ungeblockten Objektträger wurden mit einem Pro-Plate-Kultivierungsaufsatz (Grace BioLabs, USA) versehen. Die Kavitäten wurden mit je 200 µl Blocklösung gefüllt und 30 min bei Raumtemperatur inkubiert. Die Kavitäten wurden zwei mal mit PBS-Lösung gewaschen und mit biologischen Zellen besiedelt (vgl. unten).
   Beim Blockieren gemäß den Verfahren (C) und (D) entfiel das Trocknen und Scannen und es schloss sich direkt die Besiedelung der Trägerplatte mit den biologischen Zellen (Beispiel 4) an.

### Beispiel 4: Besiedeln der Trägerplatte mit biologischen Zellen

(A) Generelle Richtlinien:
   Die Zahl der Zellen, die eingesetzt werden soll, ist abhängig von der beabsichtigten Inkubationsdauer und von der Art des Assays, der im Anschluss an die Besiedelung durchgeführt werden soll. Die Zellzahl pro Mikrospot sollte im linearen Bereich, bzw. im subkonfluenten Bereich liegen und wurde in Vorversuchen optimiert. Die Zellen wurden dabei durch Schütteln des Objektträgers auf diesem verteilt.
   Das Schütteln hat zwei Funktionen: 1. Gewährleisten einer gleichmäßigen Verteilung der Zellen innerhalb der Kavitäten. Das Schütteln der Objektträger muss unmittelbar nach dem Aussäen der Zellen erfolgen, bevor die Zellen sich am Boden der Kavitäten abgesetzt haben. 2. Aufkonzentrieren der Zellen auf den Mikrospots. Zellen, die nicht auf Mikrospots gesunken sind, oder nicht adhäriert sind, werden durch das Schütteln wieder in den Überstand gebracht und haben erneut die Möglichkeit, auf einem permissiven Substrat zu adhärieren.
   Der Erfolg der Adhäsion von Zellen an Mikrospots ist abhängig von den erzeugten Scherkräften und damit der Stärke und Dauer des Schüttelns und den zeitlichen Abständen zwischen Schüttelvorgängen. Um aussagekräftige Ergebnisse im Test zu bekommen, wird deshalb die Stärke, Dauer und Häufigkeit des Schüttelns den Zelladhäsionskräften der eingesetzten Zellart und Art des Assays angepasst und dementsprechend in Vorversuchen optimiert.
   Sämtliche Pipettierschritte werden von Hand mithilfe einer Pasteurpipette und eines Pipettierballs oder Mikropipetten durchgeführt. Beim Abnehmen der Flüssigkeit aus einer Kavität muss der Boden vollständig mit Flüssigkeit bedeckt bleiben; die Kavitäten müssen einzeln nach Abnehmen der Flüssigkeit sofort wieder gefüllt werden. Besiedelte Mikrospots dürfen bis zur Fixierung der Zellen nicht trocken liegen.
(B) Zur Durchführung:
   Sämtliche nachfolgenden Schritte wurden steril bzw. im Reinraum durchgeführt.
   Die Besiedelung der nach den Beispielen 1 bis 3 hergestellten Trägerplatten bzw. Objektträger erfolgte mittels ProPlate Kultivierungsaufsätzen: Auf die bei Raumtemperatur für 1 h akklimatisierten Objektträger wurden eine Silikondichtung und ein Plastik-Kultivierungsaufsatz gelegt und mit seitlichen Schienen fixiert.

Die Kavitäten wurden dreimal 5 min mit PBS gewaschen und mit je 200 µl einer Zellsuspension (z.B. 1,5 Mal 10⁵ Zellen/ml) befüllt. Direkt nach dem Aussäen und während der weiteren Kultivierung im Brutschrank für 2 h wurden die Objektträger auf einem Variomag-Mikrotiterplattenschüttler (H+P Labortechnik, Teleshake 4, Best.Nr. 51440) im Abstand von 10 min geschüttelt (Schüttelmodus kreisförmig gegen den Uhrzeigersinn, 4 s, 750 x/min).

Die Kultivierungsaufsätze wurden entfernt, die Objektträger wurden einzeln einmal in 50-ml-Falcon-Röhrchen in Calcium- und Magnesiumionen-haltige PBS-Lösung getaucht; anschließend wurden anhaftende Zellen in 50-ml-Falcon-Röhrchen gefärbt, z.B. mit Coomassie-Brillant-Blue-Färbelösung (0,05 % w/v Coomassie Brillant Blue, 50 Vol.-% Methanol, 10 Vol.-% Eisessig, 40 Vol.-% H₂O) 10 min bei Raumtemperatur.

Die Objektträger wurden dreimal in PBS getaucht und anschließend liegend mit DAPI-Lösung (Sigma; 0,5 µg/ml in PBS; 5 min bei Raumtemperatur) überschichtet und nochmals in PBS getaucht. Die Objektträger wurden auf saugfähigem Papier kurz senkrecht stehend getrocknet und mit Mowiol bzw. ProLong (Molecular Probes P-7481) eingedeckt.

Die adhärierten Zellen wurden mittels Foto-Binokular bzw. für eine quantitative Analyse mit einem Fluoreszenzmikroskop mit Motortisch fotografiert und mithilfe von dazu hergestellten Leica Qwin Macros quantifiziert.

### Beispiel 5: Differenzielle Adhäsion von drei Zelllinien auf Mikrospots aus verschiedenen extrazellulären Matrixproteinen

In einem weiteren Experiment wurden Poly-L-Lysin und Nitrocellulose vorbeschichtete Trägerplatten mit Mikrospots aus jeweils verschiedenem ECM-Proteinen als Biomolekül gemäß Beispiel 2 beschichtet: BSA, Kollagen Typ I, Kollagen Typ II, Kollagen Typ III, Kollagen Typ V, Laminin EHS, Fibronektin, Laminin hupl (human placenta, menschliche Plazenta), Thrombospondin, Heparansulfat-Proteoglykan, Vitronektin, Tenascin, Kollagen Typ IV hupl, Kollagen Typ IV EHS, Fibronektin rec (rekombinant hergestelltes Fibronektin, EMP Genetech, Denzlingen).

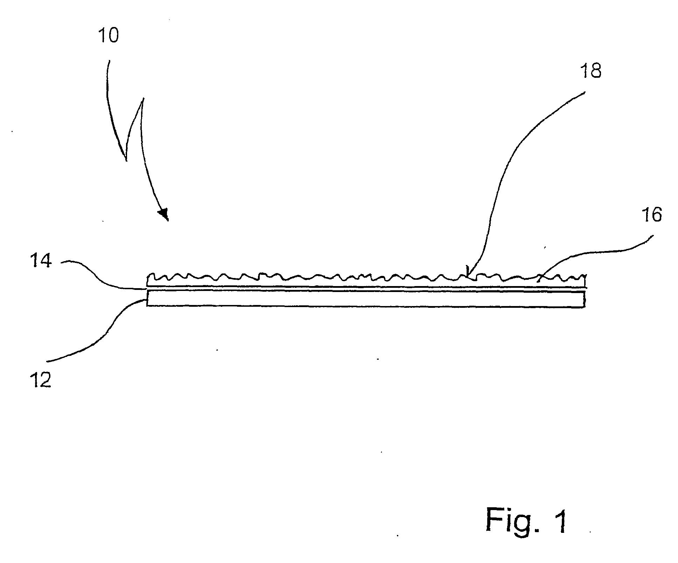

In Fig. 1 ist schematisch eine erfindungsgemäß beschichtete Trägerplatte dargestellt.

In Fig. 1 ist mit 10 insgesamt eine beschichtete Trägerplatte dargestellt. Auf einem Glasträger 12 ist eine erste Schicht 14 angebracht, bspw. eine Polykation-Schicht aus Poly-L-Lysin. Die erste Schicht 14 ist mit einer zweiten Schicht 16, die bspw. Nitrocellulose aufweist, beschichtet, welche eine raue Oberfläche 18 aufweist. Aufgrund der Rauhigkeit der Oberfläche 18 ist die Gesamtoberfläche der zweiten Schicht 16 vergrößert im Verhältnis zu einer vergleichbaren glatten Schicht. Dadurch ist es möglich, mehr Biomaterial auf die Trägerplatte 10 aufzubringen als dies bei einer glatten Oberfläche der Fall wäre. Je nach Einsatz der Trägerplatte 10 kann aber auch eine glatte Oberfläche der zweiten Schicht 16 geeignet sein, bspw. dann, wenn die Menge an aufzubringendem Biomaterial nicht so hoch sein muss oder soll wie bei einer mit einer rauen Oberfläche versehenen zweiten Schicht 16.

Die Biomoleküle wurden in je vier Mikrospots als Array von insgesamt 64 Mikrospots gedruckt. Auf dem gesamten Objektträger befanden sich insgesamt 12 Arrays. Die Arrays wurden mithilfe des Kultivierungsaufsatzes in 12 Kavitäten getrennt und konnten so unabhängig voneinander mit biologischen Zellen besiedelt werden. Zur Durchführung des Assays wurden 20,000 Zellen pro Kavität ausgesät.

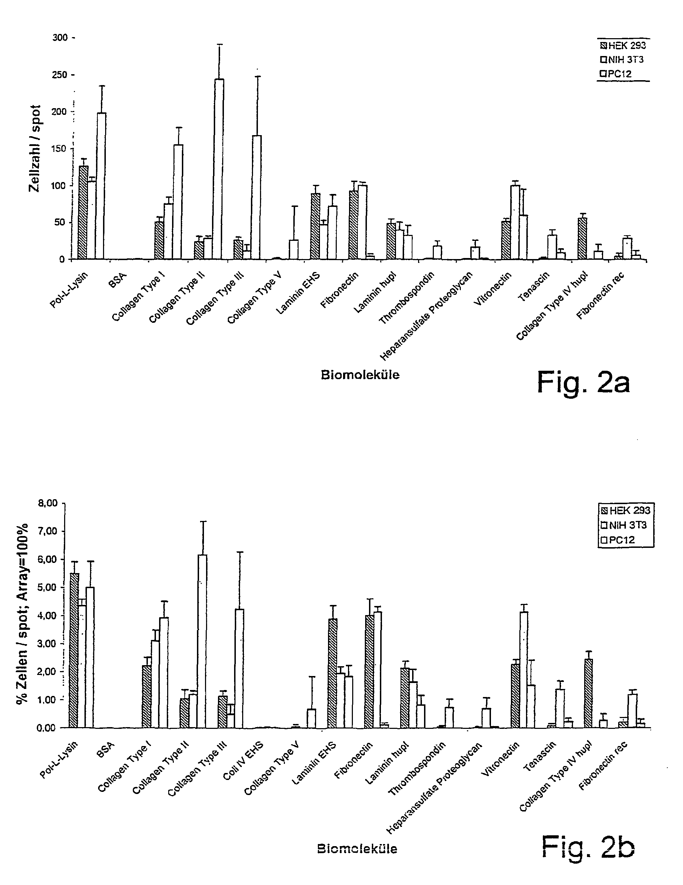

In parallelen Ansätzen wurden die Mikrospots jeweils mit den Zelllinien HEK 293, NIH 3T3 und PC 12 besiedelt, 2 h unter Schütteln kultiviert, fixiert und mit DAPI gefärbt. Anschließend wurde die Zahl der Zellen auf den Mikrospots bestimmt. Das Ergebnis eines solchen Experiments ist in den Figuren 2A und 2B dargestellt.

In Fig. 2A sind die Mittelwerte der Zahl der gebundenen Zellen der Zelllinien HEK 293 (jeweils linke Balken), NIH 3T3 (jeweils mittlere Balken) und PC 12 (jeweils rechte Balken) auf jeweils vier Mikrospots des gleichen Biomoleküls aufgetragen.

In Fig. 2B ist in analoger Weise der prozentuale Anteil der Zahl der gebundenen Zellen dargestellt, wobei eine Normierung der Zellzahl derart vorgenommen wurde, dass die Gesamtzahl der Zellen auf einem Array 100 % entspricht.

Dabei zeigte sich, dass sich je nach Zelllinientyp unterschiedliche Besiedelungsdichten für verschiedene ECM-Proteine erzielt wurden. Die erfindungsgemäß beschichtete Trägerplatte lässt sich demnach erfolgreich mit extrazellulären Matrixproteinen beschichten. Diese wiederum lassen sich je nach extrazellulärem Matrixprotein mit unterschiedlichen Zelllinien besiedeln und ergeben für jede Zelllinie ein charakteristisches Muster, durch das deren Affinität zu dem jeweiligen Protein wiedergegeben wird.

## Patentansprüche

1. Verfahren zum Beschichten einer Trägerplatte zur Durchführung funktioneller Tests an biologischen Zellen, das folgende Schritte aufweist:
(a) Bereitstellen einer Trägerplatte mit einer ersten Schicht, die Poly-L-Lysin oder Derivate davon aufweist, und
(b) Beschichten der mit der ersten Schicht beschichteten Trägerplatte mit Nitrocellulose oder Derivaten davon, zur Bildung einer rauen Nitrocellulose-Schicht mit einer Schichtdicke von ca. 100 bis ca. 1200 nm.

2. Verfahren nach Anspruch 1, **dadurch gekennzeichnet, dass** die Beschichtung in Schritt (b) durch Aufbringen einer Methanol-Nitrocellulose-Lösung und anschließendem Verdunsten lassen des Methanols erfolgt.

3. Verfahren nach Anspruch 2, **dadurch gekennzeichnet, dass** das Aufbringen der Methanol-Nitrocellulose-Lösung durch Besprühen der Trägerplatte erfolgt.

4. Verfahren nach Anspruch 2, **dadurch gekennzeichnet, dass** das Aufbringen der Methanol-Nitrocellulose-Lösung durch Eintauchen der Trägerplatte in die Lösung erfolgt.

5. Verfahren nach einem der vorhergehenden Schritte, **gekennzeichnet durch** den weiteren Schritt (c):
(c) Aufbringen von Biomolekülen auf die beschichtete Trägerplatte.

6. Verfahren nach Anspruch 5, **dadurch gekennzeichnet, dass** die Biomoleküle ausgewählt sind aus der Gruppe bestehend aus: Proteine, insbesondere Proteine der extrazellulären Matrix, wie Fibronektin, Laminin, Thrombospondin, Kollagen, Elastin, Tenascin, Vitronektin; Kohlenhydrate, insbesondere Kohlenhydrate der extrazellulären Matrix, wie Glykosaminoglykane; Proteoglykane; Lipide.

7. Verfahren nach einem der vorhergehenden Ansprüche, **gekennzeichnet durch** den weiteren Schritt (c'):
(c') Aufbringen von Testsubstanzen auf die beschichtete Trägerplatte.

8. Verfahren nach Anspruch 7, **dadurch gekennzeichnet, dass** die Testsubstanzen ausgewählt sind aus der Gruppe bestehend aus: Pharmazeutische Präparate; Antikörper; Substanzen, die die Eigenschaften der biologischen Zellen beeinflussen; Botenstoffe; Wachstumsfaktoren; Antigene; Allergene.

9. Verfahren nach einem der Ansprüche 5 bis 8, **dadurch gekennzeichnet, dass** das Aufbringen der Biomoleküle und/oder Testsubstanzen mittels kontaktlosem Drucken oder mittels Kontakt-vermitteltem Drucken erfolgt.

10. Verfahren nach Anspruch 9, **dadurch gekennzeichnet, dass** die Biomoleküle und/oder Testsubstanzen durch ein Verfahren aufgebracht werden, das ausgewählt ist aus der Gruppe umfassend die ink-jet-Technologie, das Pin und Ring Verfahren und das Split-Pin-Verfahren.

11. Verfahren nach Anspruch 9 oder 10, **dadurch gekennzeichnet, dass** die Biomoleküle und/oder Testsubstanzen in einem Druckpuffer bereitgestellt werden, der Trehalose in einer Endkonzentration von 0,1 bis 5 % (w/v), vorzugsweise 0,5 % (w/v), und NP 40 in einer Endkonzentration von 0,00001 bis 0,1 % (v/v), vorzugsweise 0,0003 bis 0,005 % (v/v), enthält.

12. Verfahren nach einem der Ansprüche 5 bis 11, **gekennzeichnet durch** den weiteren Schritt (d):
(d) Inkubation der vorbehandelten Trägerplatte mit einer Protein-Lösung, vorzugsweise einer 5 Vol.-%igen BSA-PBS-Lösung.

13. Verfahren nach einem der Ansprüche 5 bis 12, **gekennzeichnet durch** den weiteren Schritt (e):
(e) Aufbringen von biologischen Zellen auf die vorbehandelte Trägerplatte.

14. Verfahren nach einem der vorhergehenden Ansprüchen, **dadurch gekennzeichnet, dass** die in Schritt (a) bereitgestellte Trägerplatte aus einem Material gefertigt ist, das ausgewählt ist aus der Gruppe bestehend aus: Glas; Kunststoff, insbesondere Polystyrol und/oder Silikon.

15. Trägerplatte zur Durchführung funktioneller Tests an biologischen Zellen, bestehend aus einer Basisplatte, die mit einer ersten Schicht beschichtet ist, die Poly-L-Lysin oder Derivate davon aufweist, und einer darauf aufgebrachten Schicht aus Nitrocellulose oder Derivaten davon, wobei die Schicht aus Nitrocellulose oder Derivaten davon eine Schichtdicke von ca. 100 bis ca. 1200 nm aufweist.

16. Trägerplatte nach Anspruch 15, **dadurch gekennzeichnet, dass** auf die Schicht aus Nitrocellulose eine Schicht aus Biomolekülen und/oder Testsubstanzen aufgebracht ist.

17. Trägerplatte nach Anspruch 16, **dadurch gekennzeichnet, dass** die Biomoleküle ausgewählt sind aus der Gruppe bestehend aus: Proteine, insbesondere Proteine der extrazellulären Matrix, wie Fibronektin, Laminin, Thrombospondin, Kollagen, Elastin, Tenascin, Vitronektin; Kohlenhydrate, insbesondere Kohlenhydrate der extrazellulären Matrix, wie Glykosaminoglykanen; Proteoglykane; Lipide.

18. Trägerplatte nach Anspruch 16, **dadurch gekennzeichnet, dass** die Testsubstanzen ausgewählt sind aus der Gruppe bestehend aus: Pharmazeutische Präparate; Antikörper; Substanzen, die die Eigenschaften der biologischen Zellen beeinflussen; Botenstoffe; Wachstumsfaktoren; Antigene; Allergene.

19. Trägerplatte nach einem der Ansprüche 15 bis 18, **dadurch gekennzeichnet, dass** auf die Biomoleküle und/oder Testsubstanzen biologische Zellen aufgebracht sind.

20. Trägerplatte nach einem der Ansprüche 15 bis 19, **dadurch gekennzeichnet, dass** die Basisplatte aus einem Material gefertigt ist, das ausgewählt ist aus der Gruppe bestehend aus: Glas, Kunststoff, insbesondere Polystyrol und/oder Silikon.

21. Verwendung einer Trägerplatte hergestellt nach dem Verfahren gemäß einem der Ansprüche 1 bis 14, oder einer Trägerplatte gemäß einem der Ansprüche 15 bis 20, zur Durchführung funktioneller Tests an biologischen Zellen.

## Claims

1. A method for coating a support plate for carrying out functional tests on biological cells, which comprises the following steps:
(a) providing a support plate with a first layer, which comprises poly-L-lysin or derivatives thereof, and
(b) coating the support plate, coated with the first layer, with nitrocellulose or derivatives thereof, to generate a rough nitrocellulose layer with a layer thickness from approximately 100 to approximately 1200 nm.

2. The method as claimed in claim 1, **characterized in that** the coating in step (b) is carried out by applying a methanol-nitrocellulose solution and subsequently letting evaporate the methanol.

3. The method as claimed in claim 2, **characterized in that** the methanol-nitrocellulose solution is applied by spraying the support plate.

4. The method as claimed in claim 2, **characterized in that** the methanol-nitrocellulose solution is applied by immersing the support plate in the solution.

5. The method as claimed in anyone of the preceding steps, **characterized by** the further step (c):
(c) applying biomolecules onto the coated support plate.

6. The method as claimed in claim 5, **characterized in that** the biomolecules are selected from the group consisting of: proteins, in particular proteins of the extracellular matrix such as fibronectin, laminin, thrombospondin, collagen, elastin, tenascin, vitronectin; carbohydrates, in particular carbohydrates of the extracellular matrix such as glycosaminoglycans; proteoglycans; lipids.

7. The method as claimed in anyone of the preceding claims, **characterized by** the further step (c'):
(c') applying test substances onto the coated support plate.

8. The method as claimed in claim 7, **characterized in that** the test substances are selected from the group consisting of: pharmaceutical compounds; antibodies; substances which influence the properties of the biological cells; messengers; growth factors; antigens; allergens.

9. The method as claimed in anyone of claims 5 to 8, **characterized in that** the biomolecules and/or test substances are applied by means of contactless printing or by means of contact-mediated printing.

10. The method as claimed in claim 9, **characterized in that** the biomolecules and/or test substances are applied by a method which is selected from the group comprising inkjet technology, the pin-and-ring method and the split-pin method.

11. The method as claimed in claim 9 or 10, **characterized in that** the biomolecules and/or test substances are provided in a printing buffer which contains trehalose in a final concentration of from 0.1 to 5% (w/v), preferably 0.5% (w/v), and NP 40 in a final concentration of from 0.00001 to 0.1% (v/v), preferably from 0.0003 to 0.005% (v/v).

12. The method as claimed in anyone of claims 5 to 11, **characterized by** the further step (d):
(d) incubation of the pre-treated support plate with a protein solution, preferably a 5 vol. percent BSA-PBS solution.

13. The method as claimed in anyone of claims 5 to 12, **characterized by** the further step (e):
(e) applying biological cells onto the pre-treated support plate.

14. The method as claimed in anyone of the preceding claims, **characterized in that** the support plate provided in step (a) is made of a material which is selected from the group consisting of: glass; plastic, in particular polystyrene and/or silicone.

15. A support plate for carrying out functional tests on biological cells, consisting of a base plate which is coated with a first layer that comprises poly-L-lysin or derivatives thereof, and a layer of nitrocellulose or derivatives thereof, applied thereon, whereby the layer of nitrocellulose or derivatives thereof comprises a layer thickness from approximately 100 to approximately 1200 nm.

16. The support plate as claimed in claim 15, **characterized in that** a layer of biomolecules and/or test substances is applied onto the nitrocellulose layer.

17. The support plate as claimed in claim 16, **characterized in that** the biomolecules are selected from the group consisting of: proteins, in particular proteins of the extracellular matrix such as fibronectin, laminin, thrombospondin, collagen, elastin, tenascin, vitronectin; carbohydrates, in particular carbohydrates of the extracellular matrix such as glycosaminoglycans; proteoglycans; lipids.

18. The support plate as claimed in claim 16, **characterized in that** the test substances are selected from the group consisting of: pharmaceutical compounds; antibodies; substances which influence the properties of the biological cells; messengers; growth factors; antigens; allergens.

19. The support plate as claimed in anyone of claims 15 to 18, **characterized in that** biological cells are applied onto the biomolecules and/or test substances.

20. The support plate as claimed in anyone of claims 15 to 19, **characterized in that** the base plate is made of a material which is selected from the group consisting of: glass; plastic, in particular polystyrene and/or silicone.

21. The use of a support plate produced by the method as claimed in one of claims 1 to 14, or a support plate as claimed in one of claims 15 to 20, for carrying out functional tests on biological cells.

## Revendications

1. Procédé destiné à enduire une plaque support permettant la réalisation de tests fonctionnels sur des cellules biologiques, qui comprend les étapes suivantes :
(a) mise à disposition d'une plaque support avec une première couche, qui comporte de la poly-L-lysine ou un dérivé de celle-ci, et
(b) enduction de la plaque support enduite de la première couche avec de la nitrocellulose ou un dérivé de celle-ci, pour former une couche de nitrocellulose rugueuse d'une épaisseur de couche d'environ 100 à environ 1200 nm.

2. Procédé selon la revendication 1, **caractérisé en ce que** l'enduction dans l'étape (b) est réalisée par application d'une solution de méthànol-nitrocellulose puis évaporation du méthanol.

3. Procédé selon la revendication 2, **caractérisé en ce que** l'application de la solution de méthanol-nitrocellulose est réalisée par pulvérisation de celle-ci sur la plaque support.

4. Procédé selon la revendication 2, **caractérisé en ce que** l'application de la solution de méthanol-nitrocellulose est réalisée par immersion de la plaque support dans la solution.

5. Procédé selon l'une quelconque des étapes précédentes, **caractérisé par** l'étape supplémentaire (c) :
(c) application de biomolécules sur la plaque support enduite.

6. Procédé selon la revendication 5, **caractérisé en ce que** les biomolécules sont choisies dans le groupe formé par les protéines, en particulier les protéines de la matrice extracellulaire comme la fibronectine, la laminine, la thrombospondine, le collagène, l'élastine, la ténascine, la vitronectine; les glucides, en particulier les glucides de la matrice extracellulaire comme les glycosaminoglycanes ; les protéoglycanes ; les lipides.

7. Procédé selon l'une quelconque des revendications précédentes, **caractérisé par** l'étape supplémentaire (c') :
(c') application de substances à tester sur la plaque support enduite.

8. Procédé selon la revendication 7, **caractérisé en ce que** les substances à tester sont choisies dans le groupe formé par les produits pharmaceutiques ; les anticorps ; les substances modifiant les propriétés des cellules biologiques ; les messagers chimiques ; les hormones de croissance ; les antigènes ; les allergènes.

9. Procédé selon l'une quelconque des revendications 5 à 8, **caractérisé en ce que** l'application des biomolécules et/ou des substances à tester est réalisée au moyen d'une impression sans contact ou au moyen d'une impression par contact.

10. Procédé selon la revendication 9, **caractérisé en ce que** les biomolécules et/ou les substances à tester sont appliquées par un procédé qui est choisi dans le groupe formé par la technologie à jet d'encre, le procédé dit « pin and ring » (aiguille et anneau) et le procédé de la goupille fendue.

11. Procédé selon la revendication 9 ou 10, **caractérisé en ce que** les biomolécules et/ou les substances à tester sont mis à disposition dans un tampon d'impression qui contient du tréhalose en une concentration finale de 0,1 à 5 % (poids/volume), de préférence 0,5 % (poids/volume), et NP 40 dans une concentration finale de 0,00001 à 0,1 % (volume/volume), de préférence 0,0003 à 0,005 % (volume/volume).

12. Procédé selon l'une quelconque des revendications 5 à 11, **caractérisé par** l'étape supplémentaire (d) :
(d) incubation de la plaque support prétraitée avec une solution de protéine, de préférence une solution BSA-PBS à 5 % en volume.

13. Procédé selon l'une quelconque des revendications 5 à 12, **caractérisé par** l'étape supplémentaire (e) :
(e) application de cellules biologiques sur la plaque support prétraitée.

14. Procédé selon l'une quelconque des revendications précédentes, **caractérisé en ce que** la plaque support mise à disposition dans l'étape (a) est constitué d'un matériau choisi dans le groupe formé par le verre ; les matières plastiques, en particulier le polystyrène et/ou la silicone.

15. Plaque support permettant la réalisation de tests fonctionnels sur des cellules biologiques, se composant d'une plaque de base, enduite d'une première couche qui comporte de la poly-L-lysine ou un dérivé de celle-ci, et une couche de nitrocellulose ou d'un dérivé de celle-ci appliquée sur celle-ci, la couche de nitrocellulose ou d'un dérivé de celle-ci présentant une épaisseur de couche d'environ 100 à environ 1200 nm.

16. Plaque support selon la revendication 15, **caractérisée en ce qu'**une couche de biomolécules et/ou de substances à tester est appliquée sur la couche de nitrocellulose.

17. Plaque support selon la revendication 16, **caractérisée en ce que** les biomolécules sont choisies dans le groupe formé par les protéines, en particulier les protéines de la matrice extracellulaire, comme la fibronectine, la laminine, la thrombospondine, le collagène, l'élastine, la ténascine, la vitronectine ; les glucides, en particulier les glucides de la matrice extracellulaire, comme les glycosaminoglycanes ; les protéoglycanes ; les lipides.

18. Plaque support selon la revendication 16, **caractérisée en ce que** les substances à tester sont choisies dans le groupe formé par les produits pharmaceutiques ; les anticorps ; les substances modifiant les propriétés des cellules biologiques ; les messagers chimiques ; les hormones de croissance ; les antigènes ; les allergènes.

19. Plaque support selon l'une quelconque des revendications 15 à 18, **caractérisée en ce que** des cellules biologiques sont appliquées sur les biomolécules et/ou les substances à tester.

20. Plaque support selon l'une quelconque des revendications 15 à 19, **caractérisée en ce que** la plaque de base est constituée d'un matériau choisi dans le groupe formé par le verre, les matières plastiques, en particulier le polystyrène et/ou la silicone.

21. Utilisation d'une plaque support fabriquée selon le procédé selon l'une quelconque des revendications 1 à 14, ou une plaque support selon l'une quelconque des revendications 15 à 20, permettant la réalisation de tests fonctionnels sur des cellules biologiques.
